# DEMANDE DE BREVET EUROPEEN

(11) **EP 3 245 987 A1**
(43) Date de publication de la demande: **22.11.2017**
(21) Numéro de dépôt: 16181884.4
(22) Date de dépôt: 29.07.2016
(51) Int. Cl.: A61F 7/10, A61F 7/00, A61F 7/02

(54) **DISPOSITIF REFRIGERANT**

(30) Priorité: 20.05.2016 EP 16170737
(71) Demandeur: Goblet, Laurent, 5081 Saint-Denis (BE); Kinet, Virginie, 5081 Saint-Denis (BE)
(72) Inventeur: GOBLET, Laurent, 5081 Saint-Denis (BE)
(74) Mandataire: Pronovem

(57) **Abrégé**

La présente invention se rapporte à un dispositif réfrigérant dont la forme est adaptée à au moins une partie du corps humain ou animal et permettant de refroidir celle-ci, ledit dispositif comportant une enveloppe en plastique (1) permettant, avant utilisation, d'être remplie par un fluide apte à être réfrigéré, caractérisé en ce que l'enveloppe (1) est compartimentée par des soudures (4) ou collages et en ce que les divers compartiments (5) sont reliés entre eux par une partie non soudée ou non collée permettant le remplissage de l'ensemble des compartiments (5) par une ou plusieurs voies d'entrée (6) principales.

## Description

### Objet de l'invention

La présente invention se rapporte à un dispositif destiné à la régulation ou à l'abaissement de la température du corps d'un être humain ou animal.

Plus précisément, l'invention concerne un dispositif permettant la régulation de la température d'un être humain ou animal, ou le refroidissement du corps en cas de surchauffe ou encore dans le cas de traitement par cryothérapie.

Le dispositif selon l'invention peut être utilisé dans le cadre d'une activité sportive ou d'une pratique professionnelle nécessitant de travailler dans un environnement à température élevée. Il peut également être utilisé à des fins médicales ou paramédicales (brûlures) et pour abaisser la température d'au moins une partie du corps humain ou animal, pour traiter certaines douleurs ou aider à résorber des hématomes (cryothérapie).

### Etat de la technique

Dans certaines professions pratiquées dans des conditions climatiques difficiles ou dans le domaine médical, il est nécessaire de diminuer la température corporelle de personnes ou patients en situation d'hyperthermie ou de fièvre. A cette fin, il existe de nombreux dispositifs en tissus, constitués de plusieurs couches telles qu'une couche isolante associée à une couche réfrigérante et une couche améliorant le confort d'utilisation. Dans ce cas, la couche réfrigérante est conçue pour recevoir au moyen de poches ou de systèmes de fixation des éléments réfrigérés comme par exemple des glaçons.

De tels dispositifs sont notamment divulgués dans les documents CN204995643U, CN204931968U, US2010286755A1, FR3011719A1.

La fabrication de ces dispositifs se présentant sous forme de supports, couvertures, compresses ou vêtements tels que des gilets, s'avère complexe et souvent coûteuse vu la multiplicité des étapes d'assemblage et la diversité des supports.

### Buts de l'invention

La présente invention vise à fournir un dispositif simple et peu coûteux permettant de faire descendre la température corporelle de l'ensemble ou d'une partie du corps d'un être humain ou animal sur une très courte durée.

### Principaux éléments caractéristiques de l'invention

L'invention divulgue un dispositif réfrigérant dont la forme est adaptée à au moins une partie du corps humain ou animal et permettant de refroidir celle-ci, ledit dispositif comportant une enveloppe en plastique permettant, avant utilisation, d'être remplie par un fluide apte à être réfrigéré ou congelé, caractérisé en ce que l'enveloppe est compartimentée par des soudures ou collages et en ce que les divers compartiments sont reliés entre eux par une partie non soudée ou non collée permettant le remplissage de l'ensemble des compartiments par une (ou plusieurs) voie(s) d'entrée principale(s).

Les modes d'exécution préférés de l'invention comportent au moins une ou une combinaison appropriée quelconque des caractéristiques suivantes :
- le fluide est un liquide tel que l'eau ou l'eau glycolée ;
- l'enveloppe est étanche au fluide ;
- la matière plastique est une polyoléfine ou un mélange de polyoléfines, en particulier du polyéthylène ;
- le polyéthylène est un polyéthylène de basse densité, d'une densité inférieure à 0,925 g/cm³ ;
- l'enveloppe comporte un (ou plusieurs) indicateur(s) de paramètre(s) mesuré(s) pouvant être relié(s) à une (ou plusieurs) sonde(s), ladite sonde pouvant, en utilisation, être fixée sur la peau ou être en contact avec la peau pour mesurer ledit paramètre ;

- l'enveloppe comporte un indicateur de température pouvant être relié à une sonde, ladite sonde pouvant être fixée, en utilisation, sur la peau pour mesurer la température du corps ;
- le dispositif est en partie ou entièrement à usage unique ;
- le dispositif comporte des moyens de fixation ou de contact sur ou avec au moins une partie du corps humain ou animal ;
- l'enveloppe du dispositif comporte au moins une ouverture qui permet d'y introduire du liquide apte à être réfrigéré ou congelé ;
- l'ouverture comporte un (ou plusieurs) moyen(s) de fermeture.

Le dispositif de l'invention peut avantageusement se décliner en de nombreuses formes telles que :
- gilet ;
- collier;
- manchon pour poignet/ou avant-bras ;
- bonnet ;
- épaulette ;
- coudière ;
- gant ;
- cuissière ;
- genouillère ;
- chevillière ;
- chaussette ;
- couverture ;
ou une combinaison quelconque de ces éléments.

L'invention se rapporte également à un procédé de mise en oeuvre d'un dispositif réfrigérant comme décrit ci-dessus, comprenant les étapes de :
- remplir les compartiments de l'enveloppe en plastique en introduisant un fluide apte à être réfrigéré ou congelé par la ou les voies d'entrée principales ;
- placer le dispositif dans un réfrigérateur ou congélateur pendant un certain temps ;
- appliquer le dispositif sur une partie du corps humain ou animal en vue de la refroidir.

La présente invention divulgue que le dispositif peut être utilisé pour la régulation de la température d'au moins une partie du corps humain ou animal lors d'activité sportive.

La présente invention divulgue que le dispositif peut également être utilisé en cryothérapie.

### Brève description des figures

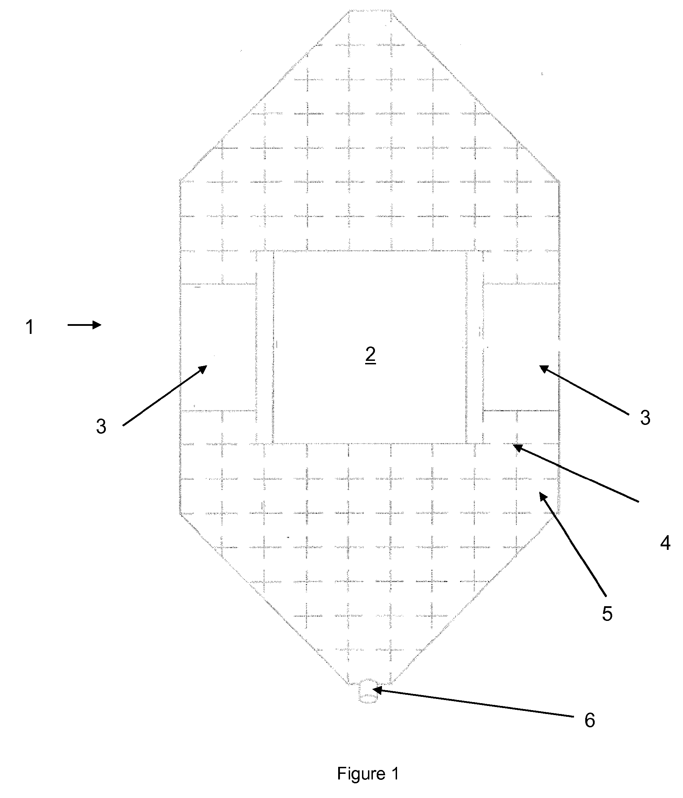

La figure 1 représente une vue schématique d'un mode de réalisation particulier du dispositif selon l'invention dans une application de gilet à effet réfrigérant.

Liste détaillée des éléments de la figure 1 :
1. Gilet à effet réfrigérant ;
2. Encolure ;
3. Partie en contact avec l'épaule ;
4. Soudure ;
5. Compartiment ;
6. Ouverture ou voie d'entrée.

### Description détaillée de l'invention

L'invention repose sur la réalisation d'un dispositif réfrigérant comportant une enveloppe permettant, avant utilisation, d'être remplie par un fluide apte à être réfrigéré ou congelé (formation de glaçons). Lorsque le dispositif réfrigérant de l'invention comportant une enveloppe contenant un fluide à réfrigérer ou à congeler est extrait du réfrigérateur, congélateur ou surgélateur, il est appliqué sur la partie du corps à refroidir.

De manière générale, le dispositif réfrigérant de l'invention est prévu pour pouvoir s'adapter à tout type de morphologie d'une partie du corps humain ou animal selon des modes de réalisation adaptés de l'invention.

L'enveloppe du dispositif selon l'invention est compartimentée. Le processus de fabrication du dispositif compartimenté selon l'invention peut être décrit de la façon suivante :
- deux feuilles de plastique sont positionnées l'une en face de l'autre ;
- les deux feuilles sont alors (pré)découpées ensemble selon un schéma prédéterminé correspondant à la morphologie d'une partie du corps humain ou animal de manière à permettre la création de compartiments de largeur et longueur spécifiques adaptés à son utilisation ;
- les deux feuilles sont ensuite (micro)soudées, thermoformées ou collées tout en laissant un espace non soudé ou non collé suffisant entre les compartiments adjacents pour laisser le liquide à réfrigérer ou à congeler s'écouler librement et remplir uniformément les compartiments.

Les soudures ou collages sont suffisamment résistantes de façon à permettre au liquide apte à être réfrigéré ou congelé de ne pas se répandre à l'extérieur de l'enveloppe en dehors de l'ouverture prévue à cet effet.

Les soudures ou collages peuvent être moins résistantes au changement de volume du liquide et se désolidariser lors de la dilatation.

Le dispositif est caractérisé en ce que l'enveloppe contenant le fluide est en matière plastique. La matière plastique est généralement une polyoléfine ou un mélange de polyoléfines, en particulier du polyéthylène de basse densité et relativement extensible.

Le dispositif comporte, avant agencement, un (ou plusieurs) indicateur(s) de paramètre(s) mesuré(s) pouvant être relié(s) à une (ou plusieurs) sonde(s). Le dispositif est muni d'un compartiment étanche (imperméable et/ou hermétique) qui n'est pas destiné à être immergé lors du remplissage des compartiments. Ce compartiment, avant fermeture par soudure, thermoformage ou collage sera rempli par un (ou des) indicateur(s) de paramètre pouvant être associé(s) à une sonde (ou plusieurs sondes) qui viendra se greffer sur ce compartiment afin d'être en contact direct avec le corps. Il s'agit, par exemple, d'un indicateur de température permettant de mesurer la variation de température, en utilisation, afin d'optimiser l'utilisation du dispositif, l'indicateur pourra être un matériau changeant de couleur ou un thermomètre digital. Au moins un élément complémentaire tel qu'un indicateur du rythme cardiaque ou un chronomètre pourra également être intégré dans ce compartiment.

La figure 1 est une vue schématique d'un mode exemplatif de réalisation du dispositif selon l'invention porté par un être humain sous la forme d'un gilet à effet réfrigérant. Celui-ci peut servir pendant ou après un effort physique. Les utilisations sont nombreuses : par exemple, un sportif peut porter ledit gilet entre deux rounds d'un match de boxe ou après un match de boxe, après une course à pied ou une course à vélo et cela afin de faciliter la régulation de la température de son corps. Ce gilet permet d'atteindre assez rapidement (en quelques minutes) la température normale du corps humain d'un adulte sain qui est de 37 ± 1° degré Celsius. Une étude est actuellement menée à l'Université de Liège. Elle vise à déterminer les effets du dispositif sur des sujets sportifs en tenant compte de deux paramètres : l'effet de bien-être et l'effet physiologique.

Le gilet à effet réfrigérant peut être adapté à tout type de morphologie d'un être humain, muni dans certains cas d'un casque de protection (tel que celui d'un boxeur, par exemple). Plus particulièrement, des modèles proportionnels aux tailles XS (extra small), S (small), M (medium), L (large), XL (extra large) et XXL(extra extra large) sont prévus afin de s'adapter aux différentes tailles de la population humaine.

## Revendications

1. Dispositif réfrigérant dont la forme est adaptée à au moins une partie du corps humain ou animal et permettant de refroidir celle-ci, ledit dispositif comportant une enveloppe en plastique (1) permettant, avant utilisation, d'être remplie par un fluide apte à être réfrigéré ou congelé, **caractérisé en ce que** l'enveloppe (1) est compartimentée par des soudures ou collages (4) et **en ce que** les divers compartiments (5) sont reliés entre eux par une partie non soudée ou non collée permettant le remplissage de l'ensemble des compartiments (5) par une ou plusieurs voies d'entrée (6) principales.

2. Dispositif selon la revendication 1, **caractérisé en ce que** le fluide est un liquide tel que l'eau ou l'eau glycolée.

3. Dispositif selon l'une quelconque des revendications précédentes, **caractérisé en ce que** l'enveloppe (1) est étanche au fluide.

4. Dispositif selon l'une quelconque des revendications précédentes, **caractérisé en ce que** la matière plastique est une polyoléfine ou un mélange de polyoléfines, en particulier du polyéthylène.

5. Dispositif selon l'une quelconque des revendications précédentes, **caractérisé en ce que** le polyéthylène est un polyéthylène de basse densité, d'une densité inférieure à 0,925g/cm³.

6. Dispositif selon l'une quelconque des revendications précédentes, **caractérisé en ce que** l'enveloppe comporte un (ou plusieurs) indicateur(s) de paramètre(s) mesuré(s) pouvant être relié à une (ou plusieurs) sonde(s), ladite sonde pouvant, en utilisation, être fixée ou en contact sur la peau ou avec la peau pour mesurer ledit paramètre.

7. Dispositif selon la revendication 6 ou selon l'une quelconque des revendications précédentes, **caractérisé en ce que** l'enveloppe comporte un indicateur de température pouvant être relié à une sonde, ladite sonde pouvant être fixée, en utilisation, sur la peau pour mesurer la température du corps.

8. Dispositif selon l'une quelconque des revendications précédentes, **caractérisé en ce qu'**il est en partie ou entièrement à usage unique.

9. Dispositif selon l'une quelconque des revendications précédentes, **caractérisé en ce qu'**il comporte des moyens de fixation ou de contact sur ou avec au moins une partie du corps humain ou animal.

10. Dispositif selon l'une quelconque des revendications précédentes, **caractérisé en ce que** l'enveloppe comporte une ou plusieurs ouvertures (6) permettant l'introduction du liquide apte à être réfrigéré ou congelé dans ladite enveloppe.

11. Dispositif selon l'une quelconque des revendications précédentes **caractérisé en ce que** cette ouverture (6) comporte un (ou plusieurs) moyen(s) de fermeture.

12. Dispositif selon l'une quelconque des revendications précédentes **caractérisé en ce qu'**il prend la forme suivante :
- gilet ;
- collier;
- manchon pour poignet/ou avant-bras ;
- bonnet ;
- épaulette ;
- coudière ;
- gant ;
- cuissière ;
- genouillère ;
- chevillière ;
- chaussette ;
- couverture ;
ou une combinaison quelconque d'un des éléments précités.

13. Procédé de mise en oeuvre d'un dispositif réfrigérant selon l'une quelconque des revendications précédentes, comprenant les étapes de :
- remplir les compartiments (5) de l'enveloppe en plastique (1) en introduisant un fluide apte à être réfrigéré ou congelé par la ou les voies d'entrée (6) principales ;
- placer le dispositif dans un réfrigérateur ou congélateur pendant un certain temps ;
- appliquer le dispositif sur une partie du corps humain ou animal en vue de la refroidir.

14. Utilisation du dispositif selon l'une quelconque des revendications précédentes, pour la régulation de la température d'au moins une partie du corps humain ou animal lors d'une activité sportive.

15. Utilisation du dispositif selon l'une quelconque des revendications précédentes, pour la cryothérapie.
